# EUROPÄISCHE PATENTSCHRIFT

(11) **EP 1 468 106 B1**
(45) Veröffentlichungstag und Bekanntmachung des Hinweises auf die Patenterteilung: **18.11.2009**
(21) Anmeldenummer: 02774574.4
(22) Anmeldetag: 06.09.2002
(51) Int. Cl.: C12P 21/02

(54) **OPTIMIERUNG DER TEMPERATURSHIFTUNG BEI HOCHZELLDICHTEFERMENTATIONEN**
OPTIMIZATION OF TEMPERATURE SHIFTING IN HIGH CELL-DENSITY FERMENTATIONS
OPTIMISATION DU CHANGEMENT DE TEMPERATURE AU COURS DE FERMENTATIONS A HAUTE DENSITE CELLULAIRE

(30) Priorität: 20.09.2001 DE 10146445
(43) Veröffentlichungstag der Anmeldung: 20.10.2004
(73) Patentinhaber: Sanofi-Aventis Deutschland GmbH, 65929 Frankfurt am Main (DE)
(72) Erfinder: MAHR, Klaus, 65239 Hochheim (DE); EISBRENNER, Guenter, 60529 Frankfurt (DE); MAERKL, Herbert, 21218 Seevetal (DE)
(86) Internationale Anmeldenummer: PCT/EP2002/009976
(87) Internationale Veröffentlichungsnummer: WO 2003/027305

(56) Entgegenhaltungen:
- WO-A-02/36746
- WO-A-96/03521
- ZABRISKIE D W ET AL: "FACTORS INFLUENCING PRODUCTIVITY OF FERMENTATIONS EMPLOYING RECOMBINANT MICROORGANISMS" ENZYME AND MICROBIAL TECHNOLOGY, Bd. 8, Nr. 12, 1986, Seiten 706-717, XP008017698 ISSN: 0141-0229
- BAUER S ET AL: "PILOT SCALE EXPONENTIAL GROWTH OF ESCHERICHIA COLI W TO HIGH CELL CONCENTRATION WITH TEMPERATURE VARIATION" BIOTECHNOLOGY AND BIOENGINEERING, INTERSCIENCE PUBLISHERS, LONDON, GB, Bd. 18, 1976, Seiten 839-846, XP002908090 ISSN: 0006-3592
- POERTNER R ET AL: "DIALYSIS CULTURES" APPLIED MICROBIOLOGY AND BIOTECHNOLOGY, SPRINGER VERLAG, BERLIN, DE, Bd. 50, Nr. 4, Oktober 1998 (1998-10), Seiten 403-414, XP002927924 ISSN: 0175-7598
- SAKAMOTO S ET AL: "Fermentation conditions for efficient production of thermophilic protease in Escherichia coli harboring a plasmid." APPLIED MICROBIOLOGY AND BIOTECHNOLOGY, Bd. 42, Nr. 4, 1994, Seiten 569-574, XP008017648 ISSN: 0175-7598
- PREUSTING HANS ET AL: "High cell density cultivation of Pseudomonas oleovorans: Growth and production of poly (3-hydroxyalkanoates) in two-liquid phase batch and fed-batch systems." BIOTECHNOLOGY AND BIOENGINEERING, Bd. 41, Nr. 5, 1993, Seiten 550-556, XP002242273 ISSN: 0006-3592
- FUCHS C ET AL: "Scale-up of dialysis fermentation for high cell density cultivation of Escherichia coli." JOURNAL OF BIOTECHNOLOGY, Bd. 93, Nr. 3, 2002, Seiten 243-251, XP002242274 ISSN: 0168-1656

## Beschreibung

Die Temperatur stellt einen wichtigen Parameter in der Bioverfahrenstechnik dar. Sie beeinflußt Wachstumsgeschwindigkeit und Stoffwechselabläufe von Mikroorganismen ( M.O.). Bei vielen Produktionsverfahren mit M.O. arbeitet man während des Prozeßablaufs mit verschiedenen Temperaturbereichen, wobei hier die Temperaturshiftung sowohl nach oben als auch nach unten erfolgen kann. So wird z. B. häufig bei Verfahren, bei denen sekundäre Stoffwechselmetaboliten erzeugt werden, mit 2 Temperaturbereichen gearbeitet. In der Wachstumsphase, wo die Mycelbildung erfolgt, arbeitet man mit optimalen Wachstumstemperaturen. Man erreicht hier maximale spezifische Wachstumsgeschwindigkeiten. In der Produktionsphase wird die jeweilige Temperatur auf die optimale Produktionstemperatur für den sek. Stoffwechselmetaboliten (z. B. Antibiotikum) um ca. 2 bis 5°C gesenkt. Man arbeitet hier jetzt mit submaximalen spezifischen Wachstumsgeschwindigkeiten. Das Mycelwachstum erreicht einen statischen Zustand, wo sich Bildungsrate und Absterberate im Gleichgewicht befinden. Mit diesen Fermentationsbedingungen lassen sich hohe Ausbeuten erzielen. Ein weiteres Beispiel, in dem mit Temperaturshiftung gearbeitet wird ist die Herstellung von Interferon (Europäisches Patent Nr. 0058687) mit diploiden humanen Zellen. Hierbei erfolgt die Temperaturshiftung sowohl nach oben als auch nach unten. In der Phase 1 erfolgt bei 34°C die Induktion der Zellen; in der Phase 2 wird zur Initiierung der Produktion von Interferon die Temperatur für etwa 2 Stunden auf 37°C hoch geshiftet und in der Phase 3 erfolgt ein Shift auf die Interferonerzeugungstemperatur von 30°C. Bei dieser Temperatur wird dann der Hauptteil des Interferonertrages erzeugt.

Temperaturshiftungen werden häufig bei Fermentationen zur Herstellung von Enzymen, zur höheren Biomasseproduktion , zur Reduzierung von unerwünschten Nebenprodukten und als Induktionsmethode angewendet. In der Bioverfahrenstechnik ist es also Stand der Technik, mit Temperaturshiftungen zu arbeiten, wobei hier die Temperaturen sehr schnell von einem Bereich in den anderen geshiftet werden. Man kann das praktisch als Sprungfunktion der Temperatur betrachten.

Sakamoto et al. (Appl. Microbiol. Biotechnol. (1994) 42; 569-574) haben Fermentationsbedingungen für eine effiziente Produktion einer thermophilen Protease in E. coli beschrieben. Die Fermentationstemperatur beeinflusste die Genexpression in der Weise, dass bei einer Temperaturemiedrigung von 37°C auf 34°C zur Zeit der Induktion mit IPTG ein 28 %iger Anstieg der Proteinexpressionsrate gegenüber einer Fermentation ohne eine solche Temperaturemiedrigung gemessen wurde. Zabriskie und Arcuri (Enzyme Microbiol. Technol. December 1986, Vol. 8, 706-717) haben Faktoren untersucht, die die Produktivität von Fermentationen mit rekombinanten Mikroorganismen beeinflussen. Es stellte sich heraus, dass es vielfältige Beziehungen zwischen der mikrobiellen Physiologie, der Plasmidanzahl, der Plasmidstabilität und der Genexpression gibt. Bauer und White (Biotechnology and Bioengineering, Vol. XVIII, (1976), 839-846) haben das exponentielle Wachstum von E. coli zu hohen Dichten bei verschiedenen Temperaturen im halbtechnischen Maßstab untersucht.

Nicht zum Erfolg führte die Temperaturshiftung nach den oben erwähnten Kenntnissen bei Fed - Batch Fermentationen mit rekombinanten E. coli, wo die Expression mit Hilfe von Induktoren, wie IPTG oder IPA eingeleitet wird.
Der Grund hierfür liegt darin, daß die submaximalen spezifischen Wachstumsgeschwindigkeiten der Stämme nicht durch die Temperatur, sondern durch die Induktion beeinflusst werden.

Die Fermentationsverfahren werden in 2 Phasen eingeteilt. Phase 1: Es ist die Wachstumsphase, wo mit der maximalen spezifischen Wachstumsgeschwindigkeit hauptsächlich die Zellmasse gebildet wird. Phase 2: Hier erfolgt nach Induktion der Übergang in die Expressions - oder Produktionsphase. Bei unverändertem Temperaturniveau verlangsamen sich unmittelbar nach der Zugabe des Induktors Atmung und Stoffwechsel und damit verbunden auch das Zellwachstum. Die Zellen befinden sich in der Phase, in der mit der submaximalen spezifischen Wachstumsgeschwindigkeit rekombinantes Protein produziert wird. Shiftet man bei diesem Zustand jetzt noch zusätzlich sehr schnell die Temperatur nach unten, kommt der gesamte Prozess zum Erliegen.

Auch bei Hochzelldichtefermentationen in 2 I Membran - Dialyse Fermentern führte die schnelle Temperaturshiftung von 35 auf 26°C nicht zum Erfolg (vergleiche Beispiele 1 und 2).

Es sind Überlegungen angestellt worden, wie man die Temperaturshiftung anders gestalten kann, z. B. die Temperaturshiftung so durchzuführen, daß sich weder bei der Biotrockenmasse (BTM)-Produktion noch bei der Fusionsprotein (FP)-Erzeugung negative Einflüsse einstellen. Da die Wachstumsgeschwindigkeit in der Induktionsphase bei der Prozessführung mit konstanter Temperatur sich stetig geringfügig verzögert, sollte im folgenden versucht werden, durch eine an die Wachstumsrate der Organismen angepasste stetige Abkühlung, die Wachstums- und Produktionsbedingungen zu optimieren. Dazu sollte die Temperatur ab 40 Minuten nach der Induktion um 1,5°C pro Stunde auf eine Endtemperatur von 26°C vermindert werden. Bei der Versuchsfermentation AD 32 (siehe Beispiel 3) wurde ein optimiertes Temperaturprofil gefunden, bei dem überraschenderweise die BTM- und der FP-Gehalt sehr hoch waren. Es wurden hier 125,9 g/l BTM und 27,9 g/l FP erzeugt. Die spez. Produktivität betrug hier 222 mg FP/g BTM.

Gegenstand der Erfindung ist demgemäß ein Verfahren zur Expression eines heterologen Proteins in E. coli Zellen unter langsamer Temperaturshiftung während der Expressionsphase, wobei die Temperatur 20 bis 60 Min., vorzugsweise 40 Min. nach der Induktion die mit einem geeigneten Induktor erfolgt in kleinen Schritten von 0.2 bis 0.8°C, vorzugsweise 0.5°C jeweils im Abstand von 10 bis 30 Min., vorzugweise von 20 Min. von 35°C auf 26°C abgesenkt wird; vorzugsweise **dadurch gekennzeichnet, daß** die Kultivation der E. coli Zellen in Membran - Dialyse Fermenter, die mehr als 2 I Inhalt haben, durchgeführt wird; ganz besonders bevorzugt **dadurch gekennzeichnet, daß** die Dialyse - Membran intern oder extern am Fermenter installiert wird; besonders bevorzugt **dadurch gekennzeichnet, daß** die Membranen einen Cut-off von mindestens 10 kDal. haben; insbesondere **dadurch gekennzeichnet, daß** die Induktion bei einer optischen Dichte von 90 bis 110, vorzugsweise bei 100 erfolgt; insbesondere besonders bevorzugt **dadurch gekennzeichnet, daß** nach erfolgter Temperaturshiftung auf 26°C diese Temperatur bis zum Kultivationsende beibehalten wird; besonders **dadurch gekennzeichnet, daß** mit einem Minimal-Medium gearbeitet wird; insbesondere **dadurch gekennzeichnet, daß** die Kohlenhydratkonzentration 0,1 bis 1,0 g/l beträgt; und speziell **dadurch gekennzeichnet, daß** die Sauerstoffsättigung >10 % beträgt.

### Beispiele

Die nachfolgenden Beispiele dienen nur zur Erläuterung des Erfindungsgedankens und sollen keineswegs einschränkend auszulegen sein.

Bei den Durchführungen der Versuchsfermentationen waren bei allen die Vorbereitungen der Fermenteranlage, das jeweilige Inoculum, das Fermentationsmedium und die Spurenelementlösung identisch.

### Vorkultur und Inocolum

Die Beimpfung der Vorkultur erfolgte unter der Sterilbank aus einer bei -80°C kryostatierten Workingseed (1 ml) in einem 500 ml Schüttelkolben mit Schikanen und mit einem Füllvolumen von 100 ml des sterilisierten Vorkulturmediums.

Ansatz bezogen auf 1 I Medium:

| | |
|---|---|
| NaCl | 3,50 g |
| Hefeextrakt | 8,00 g |
| Glucose | 1,00 g |
| K₂HPO₄ | 3,68 g |
| KH₂PO₄ in H₂O | 1,32 g |

Die Vorkultur wurde 3,25 h bei 37 °C und 200 rpm auf einem Orbitalschüttler inkubiert. Nach Entnahme von 1 ml Probe wurde mit einer Verdünnung von 1:10 die optische Dichte bei 540 nm bestimmt. Mit der gemessenen opt. Dichte errechnet sich das jeweilige Animpfvolumen.

### Fermentationsmedium

| | |
|---|---|
| Glucose - Monohydrat | 38,62 g/l |
| Zitronensäure x H₂O | 3,53 g/l |
| NaOH (33 % ig ) | 1,15 g/l |
| H₃PO₄ (85 % ig ) | 2,00 ml/l |
| K₂HPO₄ | 1,18 g/l |
| MgSO₄ x 7H₂O | 2,00 g/l |
| Na₂SO₄ | 3,00 g/l |
| Spurenelementlösung | 10,00 ml/l |
| Fe₂ (SO₄ )₃ x H₂O | 0,50 g/l |
| Desmophen in H₂O _{dest.} | 0,25 ml/l |

Der pH - Wert vor der Sterilisation wurde mit 33%iger NaOH auf 3,5 eingestellt. Nach der Sterilisation erfolgte die pH Einstellung mit 25%iger NH₃ Lösung auf 7,1. 5ml Thiamin - HCl (5g/l) wurden nach der Sterilisation zu dem Medium im Innenraum zugegeben.

### Spurenelementlösung (100 fache Konz.):

| | |
|---|---|
| H₃PO₄ | 2,000 g/l |
| CuSO₄ x 5 H₂O | 0,160 g/l |
| MnSO₄ x H₂O | 1,232 g/l |
| ZnSO₄ x 7 H₂O | 1,600 g/l |
| ( NH₄ )₆ Mo₇O₂₄ x 4 H₂O | 0,800 g/l |
| KJ | 0,400 g/l |

### Vorbereitung der Fermenteranlage

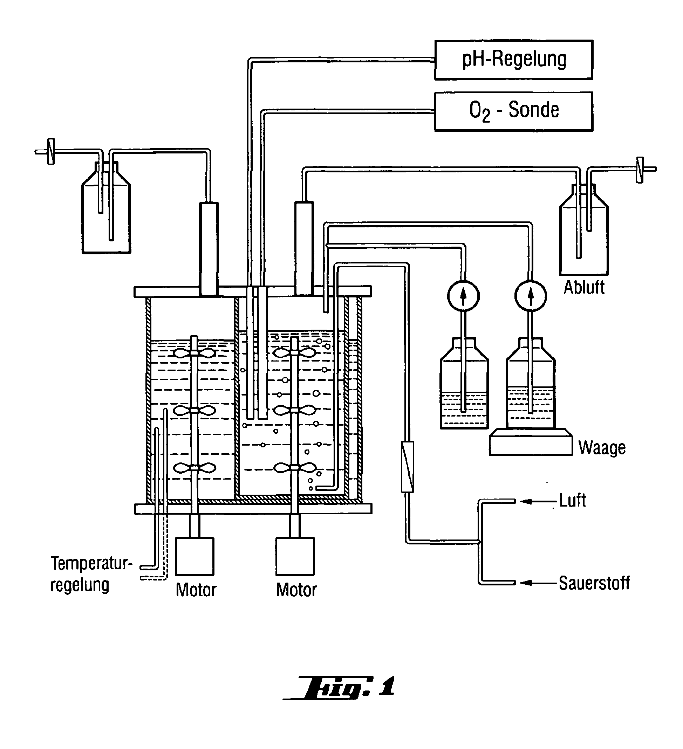

Für die Fermentation wurde ein Membranbioreaktor (Bioengineering, Wald, Schweiz) mit einer Außenfolie der Stärke 80µm (Naturin) verwendet.
Für die Trennung von Fermentations- und Dialysatraum wurde eine Cuprophan - Dialysemembran (Akzo) mit einer Wandstärke von 20 µm und einem Molekularen Cut - off von 10 kDal eingesetzt (Figur 1).

Die Sterilisierung der Anlage erfolgte insitu mit dem Fermentationsmedium bei 121°C für 20 min. Das Volumen betrug vor der Sterilisation im Innenraum 1100 ml und 3900 ml im Außenraum. Die Anschlüsse für Begasung, Abluft, Probenahme im Innen - und Außenraum sowie für die Zugabe von konzentrierter Feed - und Ammoniaklösung wurden separat im Autoklaven bei 121°C für 20 min. sterilisiert und nach der Sterilisierung des Fermenters im Flammenschatten durch Diaphragmen angeschlossen. Nach der Sterilisierung wurde aus dem Innenraum Medium abgelassen, um während der Sterilisation aus dem Außenraum in den Innenraum übergegangenes Medium auszugleichen und das Volumen wieder auf 1100 ml einzustellen. Der pH - Wert wurde mit 25%iger NH₃ - Lösung auf 7,1 eingestellt. Im Innenraum wurden 5 ml Thiamin - HCl Lösung (5 g/l) zugegeben.

### Beispiel 1 (AD 28): Versuch bei konstanter Fermentationstemperatur von 35°C

Das sterilisierte Medium wurde mit 9 ml Inoculum beimpft. Die Fermentation wurde im Batch betrieben bis die Glucosekonzentration im Fermenterinnenraum einen Wert von 3 g/l unterschritten hatte. Ab 17,31 Stunden Versuchszeit wurde die Zudosierung von konzentrierter Glucoselösung im Fermenterinnenraum mit einer peristaltischen Pumpe der Fa. Watson - Marlow gestartet. Im weiteren wurde die Glucosekonzentration im Fermenterinnenraum regelmäßig bestimmt und durch manuelle Anpassung der Pumpendrehzahl die Substratkonzentration im Bereich von 0,5 g/l Glucose geregelt.

Nach 18,3 Stunden erfolgte die Induktion bei einer optischen Dichte von OD₅₄₀ = 95,5 durch Zugabe von 3 ml 30%iger sterilfiltrierter IPTG-Lösung im Fermenterinnenraum und 10 ml 30%iger IPTG-Lösung im Fermenteraußenraum.

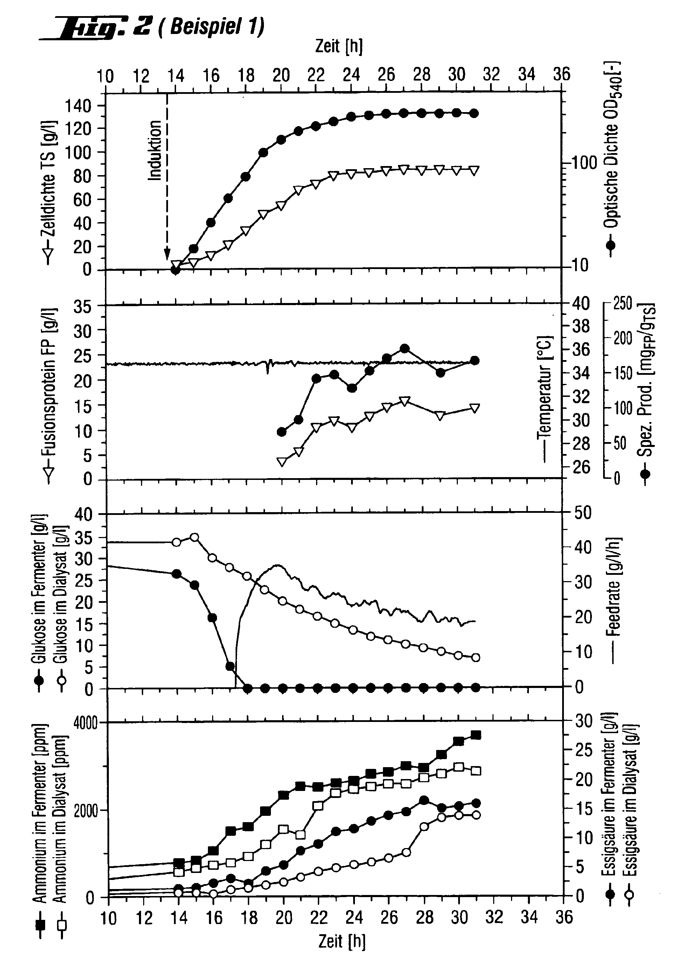

Bei einer Rührerdrehzahl von 1500 rpm im Fermenterinnenraum und 800 rpm im Fermenteraußenraum erfolgte bei 35°C und mit einer Sauerstoffsättigung von >10% die Kultivation. Die Messung des pH-Wertes erfolgte mit einer Einstabmeßkette der Fa. Broadley James, USA. Mit Hilfe eines pH-Reglers der Fa. Bioengineering und durch Zudosierung von 25%iger NH₃-Lösung mit einer peristaltischen Pumpe der Fa. Meredes, wurde der pH-Wert online auf 7,1 geregelt.
Nach 31 Stunden wurde die Fermentation beendet. Die Trockensubstanz in der 27. Stunde betrug maximal 84 g/l und die Produktkonzentration 15,8 g/l Fusionsprotein. Der zeitliche Verlauf der Fermentation ist in Figur 2 dargestellt.
Zum Zeitpunkt der Induktion war die maximale spezifische Wachstumsrate µₘₐₓ = 0,511 / h, die nach 2 Stunden Expression auf µ = 0,282 / h abgefallen ist und hiernach stetig, bis zur 27. Stunde, wo die maximale Produktivität erfolgte, sich auf µ = 0,013 / h verlangsamte.
Die maximale Acetatbildung betrug 16,5 g/l

### Beispiel 2 (AD 6): Rasche Temperaturabsenkung nach dem Stand der Technik, von 36°C auf 26°C

Das Animpfvolumen von 12 ml wurde mit einer sterilen Spitze im Flammenschatten auf den Fermenter überführt. Die Fermentation wurde im Batch betrieben bis die Glucosekonzentration im Fermenterinnenraum einen Wert von 3 g/l unterschritten hatte. Ab 15,28 Stunden Versuchszeit wurde die Zudosierung von konzentrierter Glucoselösung im Fermenterinnenraum mit einer peristaltischen Pumpe der Fa. Watson-Marlow gestartet. Im weiteren wurde die Glucosekonzentration im Fermenterinnenraum regelmäßig bestimmt und durch manuelle Anpassung der Pumpendrehzahl die Substratkonzentration im Bereich von 0,5 bis 1,0 g/l Glucose geregelt.

Nach 15,85 Stunden erfolgte die Induktion bei einer optischen Dichte von OD₅₄₀ = 92 durch Zugabe von 3,5 ml 30%iger sterilfiltrierter IPTG-Lösung im Fermenterinnenraum und 10,5 ml 30%iger IPTG-Lösung im Fermenteraußenraum.
Die Rührerdrehzahl betrug 1500 rpm im Fermenterinnenraum und 800 rpm im Fermenteraußenraum. Im Verlauf der Fermentation wurde die Rührerdrehzahl im Fermenterinnenraum auf maximal 2300 rpm gesteigert.

Bis 30 min. nach der Induktion (16,35 Stunden) betrug die Temperatur 35°C. Dann wurde der Temperatursollwert auf den Endwert von 26°C eingestellt und bis zum Ende der Fermentation konstant gehalten. Die heruntergeshiftete Temperatur von 26°C wurde nach 0,48 Stunden erreicht. Die Messung des pH-Wertes erfolgte mit einer Einstabmeßkette der Fa. Broadley. Mit Hilfe eines pH-Reglers der Fa. Bioengineering und durch Zudosierung von 25%iger NH₃-Lösung mit einer peristaltischen Pumpe der Fa. Meredes wurde der pH-Wert online auf 7,1 geregelt.

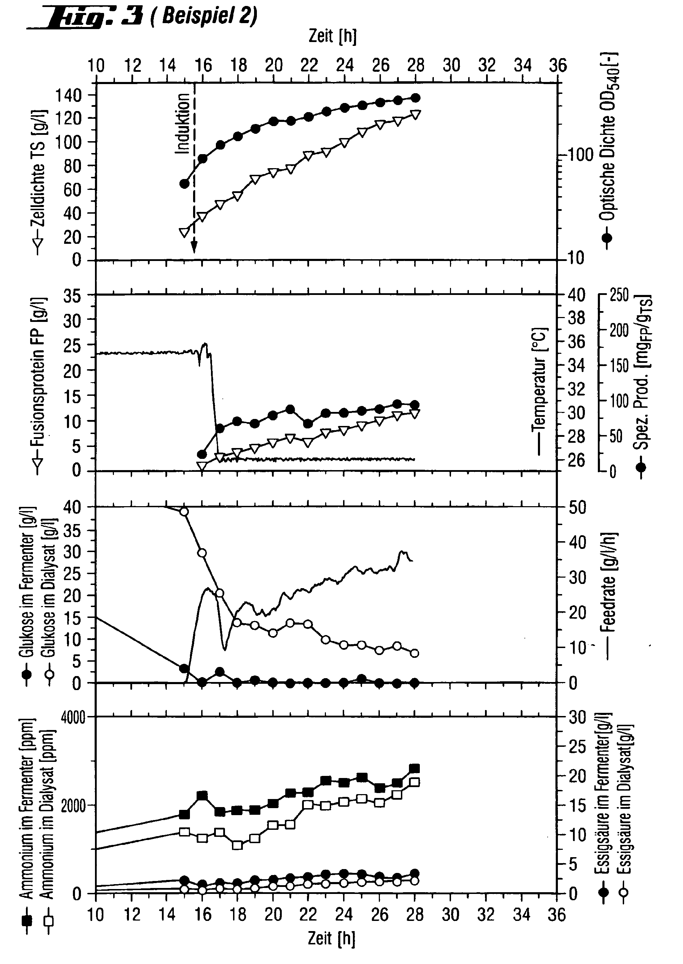

Die Konzentration des gelösten Sauerstoffs pO₂ wurde durch Mischung von Luft und O₂ bei einem Gesamtstrom von 50 I_{N}/h auf einen Wert >10% Sauerstoffsättigung geregelt. Nach 28 Stunden wurde die Fermentation beendet. Die maximale Trockensubstanz betrug 122,8 g/l und die Produktkonzentration 11,6 g/l Fusionsprotein.
Der zeitliche Verlauf der Fermentation ist in Figur 3 dargestellt.
Die maximale spezifische Wachstumsrate betrug zum Zeitpunkt der Induktion µₘₐₓ = 0,575 / h. Nach der Temperaturabsenkung auf 26°C ist sie sofort sprunghaft auf µ = 0,298 / h abgefallen. Hiernach verlangsamte sich die Wachtumsrate stetig bis Ende der Fermentation auf µ = 0,046 / h. Die Acetatkonzentration betrug 3,5 g/l.

### Vergleich der Beispiele 1 und 2:

Die in Beispiel 2 zitierte Versuchsfermentation (AD 6) erreichte zwar mit 122,8 g/l eine recht hohe Biotrockenmasse, aber der Fusionsproteingehalt war mit 11,6 g/l im Vergleich zum Beispiel 1 niedriger. Der AD 28 (siehe Beispiel 1) erreichte mit konstanter Fermentationstemperatur von 35 °C eine Biotrockenmasse von 84 g/l und ein Fusionsproteingehalt von 15,8 g/l. Ein Vergleich der spezifischen Produktivität (mg Fusionsprotein (FP)/ g Biotrockenmasse (BTM) beider Fermentationen zeigt, daß diese beim AD 6 deutlich schlechter ist. Während beim AD 28 eine spezifische Produktivität von 186 mg FP / g BTM erzeugt wurde, waren es beim AD 6 nur 95 mg FP /g BTM. Dies zeigt deutlich, daß sich die schnelle Temperaturshiftung zwar positiv auf das Zellwachstum und auf die Acetatbildung ausgewirkt hat, jedoch auf die Produktbildung einen negativen Einfluß ausübte.

### Weitergehende Versuche:

Die Versuche zum Temperatureinfluß auf die Proteinexpression in E. coli wurden in einem Labordialysereaktor der Fa. Bioengineering mit einer Außenfolie der Stärke 80 µm der Fa. Naturin verwendet. Für die Trennung von Fermentations - und Dialysatraum wurde eine Cuprophan - Dialyse - Membran mit einer Wandstärke von 20 µm und einem molekularen Cut-off von 10 kDal eingesetzt. Die Membranaustauschfläche betrug 50 m²/m³ Zellsuspension. Zu Beginn der Kultivierung wurden im Fermenterinnenraum ein Anfangsvolumen von 1,1 I und im Fermenteraußenraum 3,6 I Dialysat vorgelegt. Als zusätzliche Dialyse wurden während des Fermentationslaufs 2 Liter einfach konzentriertes Medium im Fermenteraußenraum ausgetauscht..Der Versuchsaufbau ist in Figur 1 schematisch dargestellt.

### Vorkultur und Inoculum

Die Beimpfung der Vorkultur erfolgte unter der Sterilbank aus einer bei - 80 °C kryostatisierten Workingseed (1 ml) in einen 500 ml Schüttelkolben mit Schikanen mit einem Füllvolumen von 100 ml des sterlisierten Vorkulturmediums.

Ansatz bezogen auf 1 I Medium: wie bei den Beispielen 1 und 2.

Die Vorkultur wurde 3,25 h bei 37 °C und 200 rpm auf einem Orbitalschüttler inkubiert.

Nach Entname von 1 ml Probe wurde mit einer Verdünnung von 1:10 die optische Dichte bei 540 nm bestimmt. Mit der gemessenen opt. Dichte errechnet sich das jeweilige Animpfvolumen.

Fermentationsmedium: wie bei den Beispielen 1 und 2.

Der pH-Wert vor der Sterilisation wurde mit 33%iger NaOH auf 3,5 eigestellt. Nach der Sterilisation erfolgte die pH Einstellung mit 25%iger NH₃ Lösung auf 7,1.
5ml Thiamin - HCl (5g/l) wurden nach der Sterilisation zu dem Medium im Innenraum zugegeben.

Spurenelementlösung (100 fache Konz.): wie bei den Beispielen 1 und 2.

### Vorbereitung der Fermenteranlage

Die Sterilisierung der Anlage erfolgte insitu mit dem Fermentationsmedium bei 121°C für 20 min. Das Volumen betrug vor der Sterilisation im Innenraum 1100 ml und 3900 ml im Außenraum. Die Anschlüsse für Begasung, Abluft, Probenahme im Innen - und Außenraum sowie für die Zugabe von konzentrierter Feed - und Ammoniaklösung wurden separat im Autoklaven bei 121°C für 20 min. sterilisiert und nach der Sterilisierung des Fermenters im Flammenschatten durch Diaphragmen angeschlossen. Nach der Sterilisierung wurde aus dem Innenraum Medium abgelassen, um während der Sterilisation aus dem Außenraum in den Innenraum übergegangenes Medium auszugleichen und das Volumen wieder auf 1100 ml einzustellen. Der pH - Wert wurde mit 25%iger NH₃ - Lösung auf 7,1 eingestellt. Im Innenraum wurden 5 ml Thiamin - HCl Lösung (5 g/l) zugegeben.

### Beispiel 3 (AD 32): Optimiertes Temperaturprofil

Das sterilisierte Medium wurde mit 7 ml Inoculum mittels steriler Spitze im Flammenschatten beimpft. Die Fermentation wurde im Batch betrieben bis die Glucosekonzentration im Fermenterinnenraum einen Wert von 3 g/l unterschritten hatte. Ab 18,1 Stunden Versuchszeit wurde die Zudosierung von konzentrierter Glucoselösung im Fermenterinnenraum mit einer peristaltischer Pumpe der Fa.
Watson-Marlow gestartet. Im weiteren wurde die Glucosekonzentration im Fermenterinnenraum regelmäßig bestimmt und durch manuelle Anpassung der Pumpendrehzahl die Substratkonzentration im Bereich von 0,5 bis 1,0 g/l Glucose geregelt.

Nach 18,8 Stunden erfolgte die Induktion bei einer optischen Dichte von OD₅₄₀ = 94 durch Zugabe von 3,0 ml 30%iger sterilfiltrierter IPTG-Lösung im Fermenterinnenraum und 10,0 ml 30%iger IPTG-Lösung im Fermenteraußenraum.
Die Rührerdrehzahl betrug 1500 rpm im Fermenterinnenraum und 800 rpm im Fermenteraußenraum. Im Verlauf der Fermentation wurde keine weitere Variierung der Drehzahl durchgeführt.

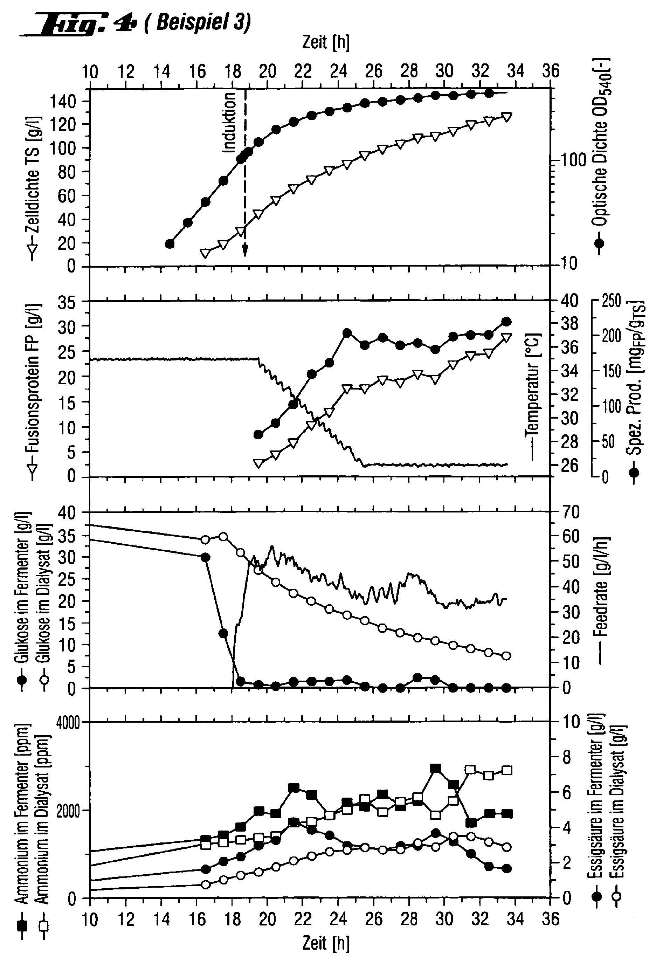

Bis 40 min. nach der Induktion (19,5 Stunden) betrug die Temperatur konstant 35°C. Dann wurde die Temperatur in Schritten von 0,5°C nach jeweils 20 min. bis auf den Endwert von 26°C eingestellt und bis zum Ende der Fermentation konstant gehalten. Die heruntergeshiftete Temperatur von 26°C wurde nach 5,7 Stunden (Laufzeit 25,2 Stunden) erreicht. Die Messung des pH-Wertes erfolgte mit einer Einstabmeßkette der Fa. Broadley.
Mit Hilfe eines pH-Reglers der Fa. Bioengineering und durch Zudosierung von 25%iger NH₃-Lösung mit einer peristaltischen Pumpe der Fa. Meredes, wurde der pH-Wert online auf 7,1 geregelt.
Die Konzentration des gelösten Sauerstoffs pO₂ wurde durch Mischung von Luft und O₂ bei einem Gesamtstrom von 50 I_{N}/h auf einenWert >10% Sauerstoffsättigung geregelt.
Nach 33,5 Stunden wurde die Fermentation beendet. Die maximale Trockensubstanz betrug 125,9 g/l und die Produktkonzentration 27,9 g/l Fusionsprotein.
Der zeitliche Verlauf der Fermentation ist in Figur 4 dargestellt.
Die maximale spezifische Wachstumsrate betrug zum Zeitpunkt der Induktion µₘₐₓ = 0,418 / h. Analog zur Temperaturabsenkung verlangsamte sich die spez. Wachstumsrate. Nach 1 Stunde Expression betrug µ = 0,342 / h. Hiernach erfolgte eine kontinuierliche Verlangsamung bis auf µ_{26°C} = 0,119/ h. Am Ende der Fermentation, nach einer Expressionsphase von 14 Stunden betrug µ = 0,016 / h.
Der Acetatgehalt lag bei 4 g/l.

### Vergleich der Ergebnisse der drei Beispiele:

Der Einfluss der Temperaturshiftung von 35°C auf 26°C auf das Wachstumsverhalten der M.O. und auf die Produktivität wurde durch die Versuchsfermentationen deutlich belegt. Eine an die spez. Wachstumsrate der M.O. angepasste langsam, stetige Temperaturshiftung hat sich sowohl auf das Wachstum als auch auf die Produktbildung positiv ausgewirkt. Es hat sich auch gezeigt, daß bei einer raschen Temperaturshiftung unmittelbar nach erfolgter Temperaturabsenkung die spezifische Wachstumsrate auch schnell um ca. die Hälfte ( µ_{35°C} = 0,575 / h auf µ_{26°C} = 0,298 / h) abnimmt. Dagegen nimmt bei langsamer Temperaturshiftung die spezifische Wachstumsrate, ähnlich wie bei Fermentationen ohne Temperaturshiftung, kontinuierlich ab. Die optimierte langsame Temperaturshiftung hat sich nicht nur positiv auf das Zellwachstum ausgewirkt, sondern die Produktbildung und die Qualität konnten deutlich verbessert werden. Es wurden bei der Versuchsfermentation 125,9 g / I BTM. und 27,9 g / I FP erzeugt, wobei sich gleichzeitig die spez. Produktqualität von ca. 180 mg FP / g BTM,
bei normalen fed - batch Fermentationen, auf 221,6 mg FP / g BTM verbesserte. Bei der raschen, dem Stand der Technik üblichen Temperaturshiftung verschlechterte sich die spez. Produktqualität auf 95 mg FP / g BTM.
Fermentationen, die bei niedrigen Temperaturen gefahren werden, produzieren weniger Nebenprodukte. Die Acetatbildung hat dies bei den Versuchsfermentationen deutlich gezeigt. 16,5 g/ I Acetat hat der Versuchsfermenter (Beispiel 1, AD 28), der mit 35°C fermentiert wurde gebildet, wo gegenüber die Fermentationen, die mit 26°C gefahren wurden, nur 3,5 g/ l (Beispiel 2, AD 6) und 4 g/ l (Beispiel 3, AD 32) erzeugten.

Die Erfindung mit der langsamen, schrittweise an das Wachstumsverhalten der M.O. angeglichene Temperaturabsenkung hat nicht zum Erliegen des Bioprozesses geführt. Das Absenken der Temperatur auf 26°C hat das Zellwachstum und den Stoffwechsel verlangsamt. Dies hat sich positiv auf die Produkt - und Acetatbildung ausgewirkt. Die Produktkonzentration konnte auf 27,9 g / l FP bei einer deutlich besseren spez. Proteinproduktivität gesteigert werden. Ein Vergleich mit technischen fed - batch Fermentationen (7 g / l) ergibt eine Vervierfachung der Ausbeuten.

## Patentansprüche

1. Verfahren zur Expression eines heterologen Proteins in E. coli Zellen unter langsamer Temperaturshiftung während der Expressionsphase, wobei die Temperatur 20 bis 60 Min., vorzugsweise 40 Min. nach der Induktion, die mit einem geeigneten Induktor erfolgt, in kleinen Schritten von 0.2 bis 0.8°C, vorzugsweise 0.5°C jeweils im Abstand von 10 bis 30 Min., vorzugweise von 20 Min. von 35°C auf 26°C abgesenkt wird.

2. Verfahren nach Anspruch 1 , **dadurch gekennzeichnet , daß** die Kultivation der E. coli Zellen in Membran - Dialyse Fermenter, die mehr als 2L Inhalt haben, durchgeführt wird.

3. Verfahren nach einem der Ansprüche 1 oder 2, **dadurch gekennzeichnet, daß** die Dialyse - Membran intern oder extern am Fermenter installiert wird.

4. Verfahren nach einem der Ansprüche 1 bis 3 , **dadurch gekennzeichnet, daß** die Membranen einen Cut-off von mindestens 10 kDal. haben.

5. Verfahren nach einem der Ansprüche 1 bis 4 , **dadurch gekennzeichnet, daß** die Induktion bei einer optischen Dichte bei 540 nm (OD₅₄₀) von 90 bis 110, vorzugsweise bei 100 erfolgt.

6. Verfahren nach einem der Ansprüche 1 bis 5, **dadurch gekennzeichnet , daß** nach erfolgter Temperaturshiftung auf 26°C diese Temperatur bis zum Kultivationsende beibehalten wird.

7. Verfahren nach einem oder mehreren der Ansprüche 1 bis 6 , **dadurch gekennzeichnet, daß** mit einem Minimal-Medium gearbeitet wird.

8. Verfahren nach einem oder mehreren der Ansprüche 1 bis 7 , **dadurch gekennzeichnet , daß** die Kohlenhydratkonzentration 0,1 bis 1,0 g/L beträgt.

9. Verfahren nach einem oder mehreren der Ansprüche von 1 bis 8 , **dadurch gekennzeichnet, daß** die Sauerstoffsättigung >10 % beträgt .

## Claims

1. A method for expressing a heterologous protein in E. coli cells while slowly shifting the temperature during the expression phase, which comprises lowering the temperature, from 20 to 60 min, preferably 40 min, after the induction, which is effected using a suitable inducer, in small steps of from 0.2 to 0.8°C, preferably of 0.5°C, in each case at intervals of from 10 to 30 min, preferably of 20 min, from 35°C down to 26°C.

2. The method as claimed in claim 1, wherein the E. coli cells are cultured in membrane dialysis fermenters which have a capacity of more than 2 1.

3. The method as claimed in either claim 1 or 2, wherein the dialysis membrane is installed internally or externally on the fermenter.

4. The method as claimed in one of claims 1 to 3, wherein the membranes have a cut-off of at least 10 kDal.

5. The method as claimed in one of claims 1 to 4, wherein the induction is effected at an optical density at 540 nm (OD₅₄₀) of from 90 to 110, preferably at 100.

6. The method as claimed in one of claims 1 to 5, wherein, after the temperature has been shifted down to 26°C, this temperature is maintained until the end of the culture.

7. The method as claimed in one or more of claims 1 to 6, wherein a minimal medium is used.

8. The method as claimed in one or more of claims 1 to 7, wherein the carbohydrate concentration is from 0.1 to 1.0 g/l.

9. The method as claimed in one or more of claims from 1 to 8, wherein the oxygen saturation is >10%.

## Revendications

1. Procédé pour l'expression d'une protéine hétérologue dans des cellules d'E. coli avec une lente évolution de la température pendant la phase d'expression, où la température est abaissée 20 à 60 min, de préférence 40 min après l'induction, qui est suivie par un inducteur approprié, en petits pas de 0,2 à 0,8°C, de préférence de 0,5°C à chaque fois à une distance de 10 à 30 min, de préférence de 20 min de 35°C à 26°C.

2. Procédé selon la revendication 1, **caractérisé en ce que** la culture des cellules d'E. coli est réalisée dans des fermenteurs à membrane de dialyse qui présentent un contenu de plus de 2 1.

3. Procédé selon l'une quelconque des revendications 1 ou 2, **caractérisé en ce que** la membrane de dialyse est installée dans ou en dehors du fermenteur.

4. Procédé selon l'une quelconque des revendications 1 à 3, **caractérisé en ce que** les membranes présentent un Cut-off d'au moins 10 kDal.

5. Procédé selon l'une quelconque des revendications 1 à 4, **caractérisé en ce que** l'induction est réalisée à une densité optique à 540 nm (OD₅₄₀) de 90 à 110, de préférence de 100.

6. Procédé selon l'une quelconque des revendications 1 à 5, **caractérisé en ce qu'**après la fin de l'évolution de la température jusqu'à 26°C, cette température est maintenue jusqu'à la fin de la culture.

7. Procédé selon l'une ou plusieurs des revendications 1 à 6, **caractérisé en ce qu'**on travaille avec un milieu minimal.

8. Procédé selon l'une ou plusieurs des revendications 1 à 7, **caractérisé en ce que** la concentration en hydrates de carbone est de 0,1 à 1,0 g/l.

9. Procédé selon l'une ou plusieurs des revendications 1 à 8, **caractérisé en ce que** la saturation en oxygène est > 10%.
